# EUROPÄISCHE PATENTSCHRIFT

(11) **EP 0 561 841 B1**
(45) Veröffentlichungstag und Bekanntmachung des Hinweises auf die Patenterteilung: **23.08.1995**
(21) Anmeldenummer: 91920829.8
(22) Anmeldetag: 03.12.1991
(51) Int. Cl.: C07D 309/06

(54) **VERFAHREN ZUR HERSTELLUNG VON FORMYLTETRAHYDROPYRANEN**
METHOD OF PREPARING FORMYLTETRAHYDROPYRANS
PROCEDE DE FABRICATION DE FORMYLTETRAHYDROPYRANNES

(30) Priorität: 14.12.1990 DE 4039918
(43) Veröffentlichungstag der Anmeldung: 29.09.1993
(73) Patentinhaber: BASF Aktiengesellschaft, 67063 Ludwigshafen (DE)
(72) Erfinder: BRENNER, Karl, D-6700 Ludwigshafen (DE); GOETZ, Norbert, D-6520 Worms 1 (DE); HENKELMANN, Jochem, D-6700 Ludwigshafen (DE); KUEKENHOEHNER, Thomas, D-6100 Frankenthal (DE); SPIEGLER, Wolfgang, D-6520 Worms 27 (DE)
(86) Internationale Anmeldenummer: EP9102289
(87) Internationale Veröffentlichungsnummer: WO9210488

(56) Entgegenhaltungen:
- EP-A- 0 219 091
- US-A- 3 555 048
- Chemical Abstracts, Band 83, no. 11, 15 September 1975, (Columbus, Ohio, US), Petrova, Z.G. et al. : "Kinetics of tetrahydrofurfural formation ", siehe Seite 800, Zusammenfassung 9653m, & Issled. Obl. Kinet., Model. Optim. ..... 1974, 2, 180- 186
- Chemical Abstracts, Band 95, no. 3, 20 Juli 1981, (Columbus, Ohio, US), Karakhanov, R.A et Al, Seite 683, Zusammenfassung 24893f

## Beschreibung

Die vorliegende Erfindung betrifft ein neues Verfahren zur Herstellung von Formyltetrahydropyranen der allgemeinen Formel I 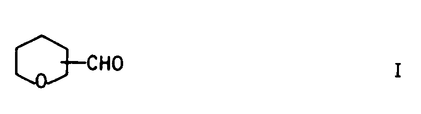
Es ist aus der US-A-2 042 220 bekannt, daß man ungesättigte primäre und sekundäre Alkohole mit einem Überschuß an Sauerstoff bei 360 bis 550°C in Gegenwart von Metallkatalysatoren, z.B. Kupfer- und Silberkatalysatoren zu den entsprechenden Aldehyden oxidieren kann. Die Katalysatoren können Legierungen, Metallverbindungen oder elementares Metall sein. Bevorzugt sind aktivierte Katalysatoren; als Aktivierungsoperationen werden eine Oberflächenamalgamierung des Metalls und anschließendes Erhitzen der Metalloberfläche angegeben. Außerdem ist der DE-B-20 41 976 zu entnehmen, daß bei dem bekannten Verfahren erhebliche Mengen an unerwünschten Nebenprodukten gebildet werden.

Die DE-A-25 17 859 beschreibt die Dehydrierung ungesättigter Alkohole an einem Kupferkatalysator, der eine Spezifische Oberfläche von 0,01 bis 1,5 m²/g hat, im wesentlichen in Abwesenheit von Sauerstoff bei 150 bis 300°C. Im Falle von α,β-ungesättigten Alkoholen als Ausgangsstoffen werden β,γ-ungesättigte Aldehyde und gesättigte Aldehyde als Nebenprodukte gebildet; die Selektivität für α,β-ungesättigte Aldehyde ist gering (Seite 2, letzter Absatz). Solche Gemische müssen in aufwendigen Trennoperationen in ihre Komponenten zerlegt werden.

In der DE-B-20 20 865 und der DE-B-20 41 976 wird die Dehydrierung von β,γ-ungesättigten Alkoholen bzw. α,β-ungesättigten Alkoholen zu α,β-ungesättigten Aldehyden beschrieben. Als Dehydrierungskatalysatoren werden auch Mischkatalysatoren, z.B. solche aus Kupfer und Silber, genannt. Nachteilig ist jedoch, daß man erhebliche Mengen an nukleophilen Substanzen zusetzen muß. Im Falle der Umsetzung von 3-Methyl-3-buten-1-ol werden gute Ergebnisse nur bei unvollständigen Umsätzen erhalten, was nach der Lehre der DE-B-22 43 810 zu Schwierigkeiten bei der Abtrennung von nicht reagiertem Ausgangsstoff führen kann.

Dehydriert man 3-Methyl-3-buten-1-ol nach dem Verfahren der DE-B-25 17 859 ohne Zusatz von Sauerstoff an metallischem Kupfer, so entstehen erhebliche Mengen an Isovaleraldehyd und die Aktivität der Katalysatoren fällt innerhalb weniger Tage rasch ab, so daß häufig regeneriert werden muß.

In der FR-A-2 231 650 wird die Herstellung von Aldehyden und Ketonen aus den entsprechenden Alkoholen durch Luftoxidation bei 250 bis 600°C in Gegenwart eines Gold-Katalysators beschrieben. Der Vorteil des Gold-Katalysators liegt in der höheren Selektivität im Vergleich zu Kupfer und Silber-Katalysatoren, so daß die Nebenprodukt-Bildung verringert wird. Nachteilig bei diesem Verfahren sind die hohen Katalysatorkosten, da ein massiver Gold-Kontakt Verwendung findet.

In der DE-B-27 15 209 und der EP-B-55 354 wird die oxidative Dehydrierung von 3-Alkyl-buten-1-olen an Katalysatoren, die aus Schichten von Silber- und/oder Kupferkristallen bestehen, unter Zusatz von molekularem Sauerstoff beschrieben. Nachteilig bei diesem Verfahren ist, daß hohe Katalysatorkosten durch Verwendung von massivem Silber auftreten und gute Selektivitäten nur erzielt werden können, wenn definierte Katalysatorkorngrößen bzw. -korngrößenverteilungen in einem Schichtenaufbau, unter Umständen sogar bestimmte Mischungen von Schichten aus Kupfer- und Silberkristallen, eingesetzt werden. Dies bedeutet sowohl eine aufwendige Füllung des Reaktors als auch eine aufwendige Katalysatorrückgewinnung. Zudem tritt bei den dabei angewandten hohen Reaktionstemperaturen Sinterung der Metallkristalle auf, was zu Druckanstieg und geringen Standzeiten führt.

In der JP-A-60/246 340 wird die Gasphasenoxidation von 3-Methyl-2-buten-1-ol zu 3-Methyl-2-buten-1-ol bei 300 bis 600°C in Gegenwart von Sauerstoff und eines Trägerkatalysators beschrieben, wobei der Katalysator jedoch auf komplizierte Weise herzustellen ist. Außerdem muß die gute Selektivität von 96,6 % durch einen geringen Umsatz erkauft werden, so daß das Verfahren für technische Zwecke kaum in Betracht kommt.

In der JP-A-58/059 933 wird die Herstellung von Aldehyden und Ketonen durch oxidative Dehydrierung von Alkoholen in Gegenwart eines Silberkatalysators, der zusätzlich Phosphor enthält, beschrieben. Um die Selektivität der Umsetzung aufrecht zu erhalten, wird in den Alkoholstrom zusätzlich eine Phosphorverbindung eingebracht, so daß man eine Verunreinigung des Produktes in Kauf nehmen muß.

Gemäß der Lehre der EP-A 263 385 können aliphatische und araliphatische primäre und sekundäre Alkohole bei erhöhter Temperatur mit Sauerstoff in Gegenwart von Kupfer-, Silber- und/oder Goldkatalysatoren zu den entsprechenden Aldehyden oder Ketonen oxidiert werden, wobei jedoch sehr spezielle Bedingungen bezüglich der Katalysatoren und des Reaktors einzuhalten sind. Außerdem wären noch höhere Umsätze wünschenswert.

Die Synthese von Formyltetrahydropyranen durch Dehydrierung der entsprechenden Hydroxymethyltetrahydrofurane ist der genannten Literatur nicht zu entnehmen.

Die bisher bekannten Verfahren zur Herstellung von Aldehyden und Ketonen sind mit Blick auf einen unkomplizierten und wirtschaftlichen Betrieb, eine lange Katalysatorstandzeit und die Selektivität der Reaktionsprozesse zur technischen Herstellung von Formyltetrahydropyranen auch wenig geeignet.

Außerdem ist die Anwendbarkeit der vorstehend genannten Oxidationsverfahren auf Hydroxymethyltetrahydropyrane äußerst zweifelhaft, da bekannterweise cyclische Ether mit Sauerstoff besonders rasch Peroxide bilden (vgl. z.B. Organikum, 2. Nachdruck der 15. Auflage, VEB Deutscher Verlag der Wissenschaften, Berlin 1981, Seite 807). Die kontinuierliche Oxidation von Hydroxymethyltetrahydropyranen in der Gasphase sollte demnach nicht oder nur mit sehr geringer Selektivität zu Formyltetrahydropyranen führen. Ferner läßt die Möglichkeit der Bildung von explosiven Nebenprodukten ein Oxidationsverfahren bei hoher Temperatur unter Verwendung von Sauerstoff technisch ungeeignet erscheinen.

Formyltetrahydropyrane werden daher üblicherweise in Lösung hergestellt, beispielsweise
1. aus Oxopyranen durch
   - Grignard-Reaktion mit Alkoxymethylmagnesiumhalogeniden, weiterer Umsetzung der Verfahrensprodukte mit Acetylchlorid oder saurer Dehydratisierung, und abschließender Hydrolyse [CA 79, 66132 g: Arm. Khim. Zh. 26, 227 (1973); CA 79, 126228 e: Dokl. Vscs. Konf. Khim. Atselinena 4th, 384 (1972); CA 76, 25029 y: Arm. Khim. Zh. 24, 503 (1971)];
   - Wittig-Reaktion mit Alkoxymethylentriphenylphosphoranen [CA 100, 68212 j: Arm. Khim. Zh. 27, 945 (1974)];
   - Überführung in Tetrahydropyranylidenglycidylester nach der Methode von Darzens und alkalischer Hydrolyse der Verfahrensprodukte [CA 87, 151967 t: Arm. Khim. Zh. 30, 516 (1977); CA 94, 30479 w: Sint. Geterotsikl. Soedin 11, 25 (1979); CA 77, 48187 k: Arm. Khim. Zh. 25, 173 (1972); CA 87, 23051 c: SU-A 550389];
2. durch Hydrierung von
   - 4-Pyranaldehyd an einem Pd/CaCO3-Katalysator, der durch Umlagerung von 4,8-Dioxabicyclo[5,1,0]-octa-2,5-dien hergestellt wurde [Angew. Chem. 86, 742 (1974];
   - Tetrahydropyran-carbonsäurechloriden nach der Methode von Rosemund an einem Pd/BaSO4-Katalysator [Collect. 7, 430 (1935)];
   - 2-phenoxy- und 2-alkoxy-substituierten 2,3-Dihydropyranen, die sich durch Cycloaddition von alkoxy- oder aryloxy-substituierten Alkenen mit 1-carboxy-2-formyl-substituierten Alkenen herstellen lassen [EP-A 219 091];
3) durch Ringerweiterung von Tetrahydro-γ-pyron mit Diazomethan und Umlagerung des Verfahrensproduktes in 4-Formyltetrahydropyran [Chem. Ber. 91, 1589 (1985)]
4) durch Behandlung von 4-Hydroxy-4-hydroxymethyl-tetrahydropyranen oder 1,6-dioxaspiro[2,5]-octanen bei erhöhter Temperatur mit einem Katalysator [DE-A 3 630 614].

Die genannten Methoden sind jedoch für eine Synthese der Formyltetrahydropyrane in technischem Maßstab wenig geeignet, da die Ausgangsverbindungen schwer handhabbar oder relativ teuer und die Ausbeuten über mehrere Verfahrensschritte unbefriedigend sind. So werden zur Herstellung von Alkoxymethyl-magnesiumhalogenid und Alkoxymethylen-triphenylphosphan Halogenmethylether benötigt, die aber wegen ihrer stark toxischen Eigenschaften unerwünscht sind.

Nach der Glycidester-Methode von Darzens erhält man substituierte 4-Formyltetrahydropyrane mit maximal 70 %iger Ausbeute. Zur Herstellung von unsubstituierten Formyltetrahydropyranen ist dieser Syntheseweg jedoch unwirtschaftlich, da die gewünschten Produkte nur in sehr geringen Ausbeuten erhalten werden.

Die Herstellung von 4-Formyltetrahydropyran durch Hydrierung von 4-Pyranaldehyd ist technisch ebenfalls unrentabel, da das Vorprodukt, also 4,8-Dioxabicyclo[5,1,0]-octa-2,5-dien nur sehr schwer zugänglich ist [vgl. Angew. Chem. 86, 742 (1974)]. Bei der in Collect. 7, 430 (1935) beschriebenen Reduktion von Tetrahydropyran-carbonsäurechloriden nach Rosemund wird für das Verfahrensprodukt ein Schmelzpunkt angegeben (135°C), wogegen 4-Formyltetrahydropyran eine bei Raumtemperatur und Normaldruck farblose, niederviskose Flüssigkeit ist. Wahrscheinlich handelt es sich bei den Feststoffen um höhermolekulare Addukte des 4-Formyltetrahydropyrans, da diese Verbindung bekanntermaßen leicht dimerisiert oder zum 4-Carboxy-tetrahydropyran autoxidiert [vgl. Chem. Ber. 91, 1589 (1985)]. Die Herstellung von 4-Formyltetrahydropyran durch Ringerweiterung von Tetrahydro-γ-pyron mit Diazomethan verläuft in sehr unbefriedigenden Ausbeuten (etwa 42 %) und ist daher für eine technische Synthese ebensowenig geeignet wie die anderen genanten Verfahren.

In einigen Fällen wurde auch die Synthese von Formyltetrahydropyranen unter oxidativen Bedingungen beschrieben, wobei jedoch immer diskontinuierlich und in Gegenwart eines inerten Lösungsmittels oder Lösungsmittelgemisches gearbeitet wird:
- Oxidation von 2-Hydroxymethyl-tetrahydropyran mit Silber-(II)-Picolinat in Dimethylsulfoxid/Wasser zu 2-Formyltetrahydropyran, wobei die Ausbeute allerdings lediglich 59 % beträgt [Canad. J. Chem. 47, 1649 (1969)];
- Oxidation von 2-Hydroxymethyl-4-methyl-tetrahydropyran mit einem Oxidationsmittel wie Chromsäure oder Pyridin-chlorchromat in beispielsweise Methylenchlorid oder Aceton zu 2-Formyl-4-methyl-tetrahydropyran [J5 4055-570].

In den Druckschriften Acta Chem. Scand., Ser. B 28 (1974), Tetrahedron Lett. 23, 4305 (1982), Carbohydr. Res. 150, 163 (1986) und Synthesis, 70 (1971) werden Verfahren zur Oxidation von Hydroxymethylgruppen in Zuckern beschrieben. Aufgrund der Ketalstruktur der Zucker können diese Methoden jedoch nicht ohne weiteres auf Hydroxymethyl-tetrahydropyrane übertragen werden.

Der Erfindung lag daher die Aufgabe zugrunde, eine einfache und technisch wirtschaftliche Methode zur Herstellung der Formyltetrahydropyrane I bereitzustellen.

Demgemäß wurde ein Verfahren zur Herstellung von Formyltetrahydropyranen der Formel I gefunden, welches dadurch gekennzeichnet ist, daß man ein Hydroxymethyltetrahydropyran der allgemeinen Formel II 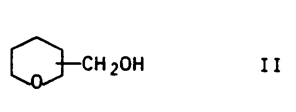
in Gegenwart eines Kupfer-, Silber- und/oder Goldkatalysators dehydriert.

Die als Ausgangsstoffe dienenden 2-Hydoxymethyl-, 3-Hydroxymethyl- und 4-Hydroxymethyltetrahydropyrane II sind z.B. durch Reduktion der entsprechenden Tetrahydropyran-carbonsäureester nach bekannten Methoden herstellbar.

Die Dehydrierung kann unter reduktiven Bedingungen oder bevorzugt unter oxidativen Bedingungen, erfolgen. Als Oxidationsmittel eignet sich insbesondere Sauerstoff, in reiner Form oder bevorzugt als Mischung mit inerten Gasen wie Stickstoff, Argon und Kohlendioxid, wobei sich ein Mischungsverhältnis von 5 bis 50 mol-% Sauerstoff auf ein Mol Inertgas empfiehlt. Zur Erhöhung der Selektivität kann es vorteilhaft sein, Wasserdampf als Inertgas oder als Bestandteil des Inertgases zu verwenden.

Besonders bevorzugt verwendet man atmosphärischen Luftsauerstoff als Oxidationsmittel.

Die Menge an Oxidationsmittel ist nicht kritisch; für eine vollständige Umsetzung werden mindestens 0,5 mol Sauerstoff (O₂) pro mol Hydroxymethyltetrahydropyran II benötigt. Bevorzugt verwendet man Mengen von 50 bis 400 mol-% an Sauerstoff, sofern sich nicht zur Erhöhung der Selektivität eine geringere Sauerstoffmenge zwischen etwa 20 und 50 mol-% empfiehlt.

Arbeitet man unter reduktiven Bedingungen ohne Sauerstoff, so empfiehlt sich eine Reaktionsführung in Gegenwart von geringen Mengen an Wasserstoff, bis etwa 0,6 mol pro mol II, um Desaktivierung des Katalysators zu vermeiden.

Für die Dehydrierung geeignete Katalysatoren sind Kupfer, Silber, Gold oder Legierungen oder Mischungen dieser Metalle. Besonders bevorzugt sind silberhaltige Katalysatoren, insbesondere Katalysatoren aus Steatit mit einer Silber-Oberfläche. Auch Mischungen der genannten Metalle mit weiteren für die Hydrierung inerten Stoffen sind möglich.

Der Katalysator kann sowohl in metallischer Form als auch an einen inerten Träger wie Kieselgel, Aluminiumoxid und Tonerde gebunden vorliegen.

Der Katalysatorträger kann auch noch inerte Zusatzstoffe enthalten, beispielsweise basische Bestandteile wie Alkalimetall- und Erdalkalimetalloxide, insbesondere Natrium- und Kaliumoxid, Alkalimetall- und Erdalkalimetallhydrogencarbonate, -acetate und -benzoate. Bevorzugt wird ein Katalysatorträger, der hauptsächlich aus Siliciumdioxid besteht.

Zweckmäßigerweise verwendet man die metallischen Katalysatoren in Form von elektrolytisch abgeschiedenen Kristallen, wobei die Korngröße der Kristalle bevorzugt zwischen 0,1 und 10 mm liegt.

Bei Katalysatoren, die an einen inerten Träger gebunden sind, empfehlen sich Schichtdicken zwischen 5 und 25 »m an Katalysatormaterial, wobei der mittlere Teilchendurchmesser besonders bevorzugt 1,6 bis 2,0 mm beträgt.

Im allgemeinen arbeitet man bei Temperaturen zwischen 180 und 750°C, besonders zwischen 200 und 600°C. Bei der Verfahrensführung unter reduktiven Bedingungen sind Temperaturen zwischen 200 und 280°C bevorzugt; bei der oxidativen Verfahrensvariante sind dagegen Temperaturen zwischen 350 und 600°C besonders vorteilhaft.

Besondere Bedingungen bezüglich des Druckes sind nicht erforderlich; normalerweise nimmt man die Umsetzung daher bei Atmosphärendruck vor.

Das erfindungsgemäße Verfahren kann sowohl diskontinuierlich als auch kontinuierlich durchgeführt werden. Bei der kontinuierlichen Arbeitsweise leitet man die Reaktionspartner über ein Wirbelbett aus einem sehr feinkörnigem Katalysator oder vorzugsweise über ein Katalysatorfestbett, wobei die Schichtdicke 5 bis 30 cm, insbesondere 10 bis 20 cm, beträgt.

In einer bevorzugten Ausführungsform verdampft man das Hydroxymethyltetrahydropyran II in einem Inertgasstrom, z.B. Stickstoff, Argon oder Kohlendioxid, erhitzt das Gasgemisch auf eine Temperatur, die ca. 100 bis 250°C unter der Reaktionstemperatur liegt und leitet es anschließend in die Reaktionszone, wobei die Kontaktzeit vorteilhaft zwischen 0,001 und 5 sec., insbesondere zwischen 0,01 und 0,2 sec. liegt.

Das gasförmige Umsetzungsprodukt kann durch Einsprühen von Lösungs- oder Verdünnungsmitteln, z.B. Kohlenwasserstoffen wie n-Hexan und Toluol, Ethern wie Methyl-tert.-Butylether oder Formamiden wie Dimethylformamid, abgekühlt und kondensiert werden.

Um eine Dimerisierung der Formyltetrahydropyrane zu vermeiden, ist es vorteilhaft, das gasförmige Umsetzungsprodukt über eine schwache, gewünschtenfalls feste Base zu leiten oder es in ein stabilisierendes Medium, beispielsweise in Wasser, ein mit Wasser mischbares organisches Lösungsmittel oder bevorzugt in eine wäßrige Lösung einer schwachen Base einzuleiten.

Als schwache Basen kommen hierbei insbesondere Erdalkalimetallhydrogencarbonate wie Natrium- und Kaliumhydrogencarbonat, Erdalkalimetallacetate wie Natrium- und Kaliumacetat sowie Erdalkalimetallbenzoate wie Natrium- und Kaliumbenzoat in Betracht.

Die Aufarbeitung des Reaktionsgemisches erfolgt wie üblich, so daß sich nähere Ausführungen hierüber erübrigen.

Geringe Verunreinigungen der Rohprodukte resultieren hauptsächlich aus der Bildung von Nebenprodukten, beispielweise durch Eliminierung von Wasser unter Bildung von Methylen-tetrahydropyranen oder durch Öffnung des Tetrahydropyran-Ringes.

Die nach dem erfindungsgemäßen Verfahren auf einfache Weise mit guter Selektivität in hohen Ausbeuten herzustellenden Formyltetrahydropyrane I sind wertvolle Zwischenprodukte zur Synthese von Pflanzenschutzmitteln und Pharmaka. Insbesondere sind sie wichtige Ausgangsprodukte zur Synthese von herbiziden oder das Pflanzenwachstum regulierenden Cyclohexan-1,3-dionen, wie sie beispielweise in der EP-A-070 370 und der EP-A-142 741 beschrieben werden. Ein brauchbarer Syntheseweg zu 5-Tetrahydropyranyl-cyclohexan-1,3-dionen, ausgehend von den Formyltetrahydropyranen I, ist z.B. aus der EP-A 352 465 und EP-A 124 041 ersichtlich.

### Beispiel 1

In ein Rohr von 1,2 cm Durchmesser wurde eine 10 cm dicke Katalysatorschicht aus Steatitkugeln, die mit 4,2 Gew.-% Silber belegt waren, eingebracht. Durch diese Katalysatorschicht leitete man bei 450°C und Atmosphärendruck kontinuierlich ein Gasgemisch, das pro Stunde aus 33,2 g (0,286 mol) 4-Hydroxymethyltetrahydropyran, 46 Nl*⁾ (2,05 mol) Stickstoff und 46 Nl Luft bestand. Nach Durchströmen der Reaktionszone wurde das gasförmige Reaktionsgemisch auf 20 bis 25°C gekühlt und in Wasser, das zur Stabilisierung des Produktes 1 Gew.-% Natriumhydrogencarbonat enthielt, eingeleitet. Die wässrige Phase wurde wie üblich aufgearbeitet. Umsatz: >99 %; Aldehyd-Selektivität: 80 %.
*⁾ Normliter

### Beispiel 2

Analog Beispiel 1 wurde ein Gasgemisch mit einer Zusammensetzung pro Stunde von 55,5 g (0,478 mol) 4-Hydroxymethyltetrahydropyran, 78 Nl (3,48 mol) Stickstoff und 78 Nl Luft umgesetzt. Umsatz: >99 %; Aldehyd-Selektivität: 83 %.

### Beispiel 3

Analog Beispiel 1 wurde ein Gasgemisch mit einer Zusammensetzung pro Stunde von 33,7 g (0,290 mol) 3-Hydroxymethyltetrahydropyran, 45,5 Nl (2,03 mol) Stickstoff und 45,5 Nl Luft umgesetzt. Umsatz: 94 %; Aldehyd-Selektivität: 90 %.

### Beispiel 4

Ein kontinuierlicher Gasstrom, der sich pro Stunde aus 24 g (0,21 mol) 4-Hydroxymethyltetrahydropyran, 90 Nl (4,02 mol) Stickstoff und 30 Nl Luft zusammensetzte, wurde bei 380°C und Atmosphärendruck durch ein zylindrisches Reaktionsrohr (Volumen 0,1 l) geleitet, das mit Raschig-Ringen aus Kupfer gefüllt war. Nach Durchströmen der Reaktionszone wurde das gasförmige Reaktionsgemisch auf 20 bis 25°C gekühlt und wie üblich auf das Produkt hin aufgearbeitet. Umsatz: 54 %; Aldehyd-Selektivität: 89 %.

### Beispiel 5

Für diesen Versuch wurde ein strangförmiger Katalysator verwendet, der aus Kieselgel mit einer Beschichtung aus 15 Gew.-% Kupfer und 1,2 Gew.-% Natriumoxid bestand. Man leitete bei 220°C und Atmosphärendruck einen kontinuierlichen Gasstrom, der sich pro Stunde aus 100 g (0,86 mol) 4-Hydroxymethyltetrahydropyran, 200 Nl (8,93 mol) Stickstoff und 10 Nl (0,45 mol) Wasserstoff zusammensetzte, durch ein zylindrisches Reaktionsrohr (Volumen 1,0 l), das mit 10 mm langen Strängen (Durchmesser 4 mm) des Katalysators gefüllt war. Nach Durchströmen der Reaktionszone wurde das gasförmige Reaktionsgemisch auf 20 bis 25°C gekühlt und in eine 1 gew.-%ige wäßrige Kaliumacetatlösung eingeleitet. Anschließend arbeitete man die wäßrige Phase wie wie üblich auf das Produkt hin auf.
Umsatz: 53 %; Aldehyd-Selektivität: 94 %.

Nach 240 Betriebsstunden war noch keine Alterung des Katalysators bezüglich Umsatz und Aldehyd-Selektivität festzustellen.

## Patentansprüche

1. Verfahren zur Herstellung von Formyltetrahydropyranen der allgemeinen Formel I 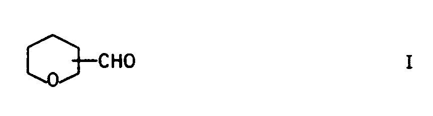 dadurch gekennzeichnet, daß man ein Hydroxymethyltetrahydropyran der allgemeinen Formel II 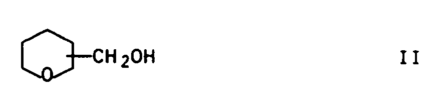 in Gegenwart eines Kupfer-, Silber- und/oder Goldkatalysators dehydriert.

2. Verfahren nach Anspruch 1, dadurch gekennzeichnet, daß man die Dehydrierung unter reduktiven Bedingungen vornimmt.

3. Verfahren nach Anspruch 1, dadurch gekennzeichnet, daß man die Dehydrierung unter oxidativen Bedingungen vornimmt.

4. Verfahren nach Anspruch 3, dadurch gekennzeichnet, daß man als Oxidationsmittel Sauerstoff oder Luft verwendet.

5. Verfahren nach den Ansprüchen 1 bis 4, dadurch gekennzeichnet, daß man die Dehydrierung bei Temperaturen zwischen 180 und 750°C vornimmt.

6. Verfahren nach den Ansprüchen 1 bis 5, dadurch gekennzeichnet, daß man das Verfahrensprodukt durch einleiten in Wasser, gewünschtenfalls in Gegenwart einer schwachen Base, stabilisiert.

7. Verfahren nach den Ansprüchen 1 bis 6, dadurch gekennzeichnet, daß der Katalysator in metallischer Form oder an einen inerten Träger gebunden vorliegt.

8. Verfahren nach den Ansprüchen 1 bis 7, dadurch gekennzeichnet, daß der Katalysator einen Siliziumdioxid-haltigen Träger enthält.

9. Verfahren nach Anspruch 7 oder 8, dadurch gekennzeichnet, daß der Katalysator-Träger eine schwache Base enthält.

## Claims

1. A process for the preparation of formyltetrahydropyrans of the formula I 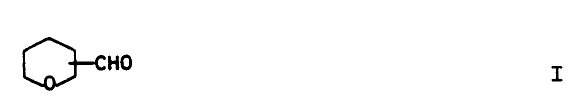 which comprises dehydrogenating a hydroxymethyltetrahydropyran of the formula II 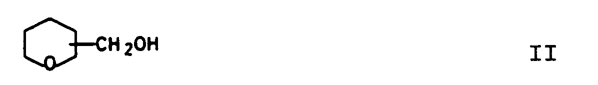 in the presence of a copper, silver and/or gold catalyst.

2. A process as claimed in claim 1, wherein the dehydrogenation is carried out under reductive conditions.

3. A process as claimed in claim 1, wherein the dehydrogenation is carried out under oxidative conditions.

4. A process as claimed in claim 3, wherein the oxidant used is oxygen or air.

5. A process as claimed in any of claims 1 to 4, wherein the dehydrogenation is carried out at from 180 to 750°C.

6. A process as claimed in any of claims 1 to 5, wherein the product is stabilized by being passed into water, if desired in the presence of a weak base.

7. A process as claimed in any of claims 1 to 6, wherein the catalyst is in metallic form or is bonded to an inert carrier.

8. A process as claimed in any of claims 1 to 7, wherein the catalyst contains a silica-containing carrier.

9. A process as claimed in claim 7 or 8, wherein the catalyst carrier contains a weak base.

## Revendications

1. Procédé de fabrication de formyltétrahydropyranes de formule générale I 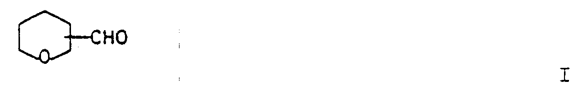 caractérisé par le fait qu'on déshydrate un hydroxyméthyltétrahydropyrane de formule générale II 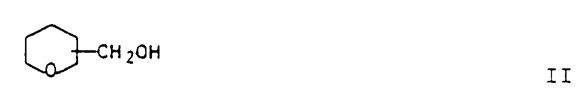 en présence d'un catalyseur de cuivre argent et/ou or.

2. Procédé selon la revendication 1, caractérisé en ce que l'on effectue la déshydratation dans des conditions réductrices.

3. Procédé selon la revendication 1, caractérisé en ce que l'on effectue la déshydratation dans des conditions oxydantes.

4. Procédé selon la revendication 3, caractérisé en ce que l'on utilise comme agent oxydant l'oxygène ou l'air.

5. Procédé selon les revendications 1 à 4, caractérisé en ce que l'on effectue la déshydratation à des températures comprises entre 180 et 750°C.

6. Procédé selon les revendications 1 à 5, caractérisé en ce que le produit obtenu par le procédé est stabilisé par introduction dans l'eau, si on le souhaite en présence d'une base faible.

7. Procédé selon les revendications 1 à 6, caractérisé en ce que le catalyseur se présente sous forme métallique ou lié à un support inerte.

8. Procédé selon les revendications 1 à 7, caractérisé en ce que le catalyseur contient un support contenant du dioxyde de silicium.

9. Procédé selon la revendication 7 ou 8, caractérisé en ce que le support de catalyseur contient une base faible.
